(19) Europäisches Patentamt
European Patent Office
Office européen des brevets

(11) Numéro de publication : 0 015 221 B1

# (12) FASCICULE DE BREVET EUROPÉEN

(45) Date de publication du fascicule du brevet : 02.12.81

(51) Int. Cl.³ : C 07 C 50/04, C 07 C 46/08

(21) Numéro de dépôt : 80420022.8

(22) Date de dépôt : 15.02.80

## (54) Procédé de préparation de para-benzoquinones.

(30) Priorité : 21.02.79 FR 7905105

(43) Date de publication de la demande : 03.09.80 (Bulletin 80/18)

(45) Mention de la délivrance du brevet : 02.12.81 Bulletin 81/48

(84) Etats contractants désignés : AT BE CH DE GB IT LU NL SE

(56) Documents cités :
EP - A - 0 001 199
FR - A - 2 138 030
FR - A - 2 319 617

(73) Titulaire : RHONE-POULENC INDUSTRIES
22, avenue Montaigne
F-75008 Paris (FR)

(72) Inventeur : Costantini, Michel
9, place Aristide Briand
F-69003 Lyon (FR)
Inventeur : Jouffret, Michel
Le Grillon Avenue du Chater
F-69340 Francheville-le-bas (FR)

(74) Mandataire : Rioufrays, Roger et al
RHONE-POULENC INDUSTRIES Centre de Recherches des Carrières Service Brevets
F-69190 Saint-Fons (FR)

EP 0 015 221 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

# Procédé de préparation de para-benzoquinones

La présente invention a pour objet un procédé de préparation de para-benzoquinones par oxydation du phénol ou des phénols substitués en ortho et/ou méta par l'oxygène ou un gaz en contenant.

Les para-benzoquinones et en particulier la p-benzoquinone non substituée — que l'on désignera plus simplement ci-après benzoquinones — sont des produits organiques particulièrement importants du point de vue industriel puisqu'ils permettent d'accéder par hydrogénation aux hydroquinones correspondantes et notamment à l'hydroquinone non substituée utilisée dans l'industrie photographique.

On a proposé de nombreux procédés de préparation de l'hydroquinone à partir du phénol ; ces procédés mettent en jeu l'hydroxylation du phénol essentiellement par l'eau oxygénée elle-même ou par des peracides organiques tels que les acides peracétique et performique engendrés « in situ » à partir de l'eau oxygénée et de l'acide carboxylique. Ils conduisent dans tous les cas à la formation concomitante d'hydroquinone et de pyrocatéchine, ce dernier produit étant généralement formé en quantités prépondérantes. Certains de ces procédés d'hydroxylation par l'eau oxygénée se sont révélés d'un grand intérêt et font l'objet d'une exploitation industrielle pour la production des diphénols. Néanmoins l'industrie est à la recherche d'un procédé permettant d'obtenir sélectivement l'hydroquinone à partir du phénol, la formation des autres diphénols et notamment de la pyrocatéchine étant limitée voire même totalement évitée. L'oxydation sélective du phénol en para-benzoquinone apparaît comme un moyen de résoudre le problème et c'est pourquoi on assiste à un effort de recherche de l'industrie pour la mise au point d'un procédé d'oxydation du phénol en benzoquinone par l'oxygène moléculaire ou un gaz en contenant.

Ainsi, dans la demande de brevet français publiée sous le numéro 2 245 602, on a décrit un procédé de préparation de benzoquinones à partir de divers phénols et notamment de la benzoquinone à partir du phénol par oxydation par l'oxygène moléculaire ou un gaz qui en contient (air par exemple), en présence d'un catalyseur comprenant du cuivre et un ligand chlore, brome, iode, thiocyanate, cyanate ou cyanure, dans un solvant polaire. Bien que le cuivre métallique puisse être mis en œuvre dans des conditions qui permettent son oxydation en ions cuivreux ou cuivriques, ce sont généralement des sels de cuivre qui sont utilisés et notamment des halogénures cuivreux ou cuivriques. Dans le brevet américain numéro 3 987 068 on a proposé un procédé analogue selon lequel la réaction est conduite en présence d'un sel de cuivre dans un nitrile formant un complexe avec le sel de cuivre. Bien que ces procédés assurent généralement un bon taux de conversion du phénol et des rendements industriellement intéressants en benzoquinone, on a constaté que lorsqu'on opère dans un appareillage qui n'est pas inerte vis-à-vis du milieu réactionnel — appareil en acier ou en fer — ses parois sont rapidement attaquées à un degré tel qu'il est pratiquement impossible d'envisager la mise en œuvre industrielle d'un tel procédé. D'autre part, on a constaté que, si l'on utilise un appareillage inerte vis-à-vis du milieu réactionnel, les rendements en benzoquinone sont limités à une valeur de l'ordre de 50 % lorsqu'on conduit la réaction en augmentant la concentration du phénol dans le milieu solvant. Il s'agit là d'un inconvénient qui va se traduire lors des opérations industrielles par une limitation sensible de la productivité de l'appareillage.

La présente invention concerne l'oxydation du phénol ou des phénols substitués en ortho et/ou en méta en benzoquinones dans un appareillage inerte vis-à-vis du milieu réactionnel et qui procure des rendements supérieurs en benzoquinone lorsque la concentration du composé phénolique dans le milieu solvant est élevée.

Plus spécifiquement la présente invention a pour objet un procédé de préparation de benzoquinones par oxydation du phénol ou des phénols mono ou polysubstitués en ortho et/ou méta par l'oxygène moléculaire ou un gaz qui en contient en opérant dans un solvant organique en présence d'ions cuivreux ou cuivriques, caractérisé en ce que l'on opère dans une cétone comme solvant en présence, en outre, d'ions carboxylates ou phénates.

Les phénols substitués qui peuvent être utilisés dans la présente invention sont ceux répondant à la formule générale :

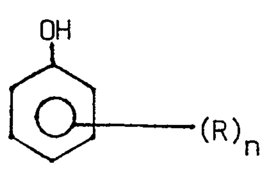

dans laquelle :

— R qui est situé en ortho ou méta par rapport au groupe hydroxyle représente : un radical alkyle comportant de 1 à 6 atomes de carbone, tel que par exemple un radical méthyle, éthyle, n-propyle, isopropyle, n-butyle, isobutyle, t-butyle, n-pentyle, isopentyle, tertioamyle, n-hexyle (éventuellement substitué par un radical alcoxy comportant de 1 à 4 atomes de carbone) ; un radical aryle comportant de 6 à 15 atomes de carbone comme un radical phényle, tolyle, xylyle ; un radical aryalalkyle comportant de 1

à 3 atomes de carbone dans le reste alkyle et 1 ou 2 noyaux benzéniques (condensés ou non) dans le reste aryle comme un radical benzyle, p-méthylbenzyle, phénéthyle ;

— deux substituants R en position ortho et méta peuvent former ensemble un radical unique divalent alkylène ou alcénylène comportant de 5 à 7 atomes de carbone et le cas échéant 1 à 2 doubles liaisons éthyléniques (ledit radical étant éventuellement substitué par 1 à 3 radicaux alkyles ou alkoxy ayant de 1 à 4 atomes de carbone) comme par exemple les radicaux tétraméthylène, butadiénylène ;

— n représente un nombre entier égal à 1, 2 ou 3.

Des exemples de phénols qui peuvent être utilisés dans la présente invention comprennent, outre le phénol lui-même : l'ortho-crésol, le méta-crésol, le diméthyl-3,5 phénol, le diméthyl-2,5 phénol, le diméthyl-2,3 phénol, le diéthyl-2,5 phénol, le diéthyl-3,5 phénol, le triméthyl-2,3,5 phénol, le tertiobutyl-2 phénol, le ditertiobutyl-2,5 phénol, l'orthophénylphénol, l'orthobenzylphénol, l'α-naphtol.

Le solvant utilisé pour conduire le procédé selon la présente invention est une cétone. Comme cétones utilisables on peut mentionner par exemple : les cétones aliphatiques, saturées ou éthyléniques, ayant de 3 à 10 atomes de carbone ; les cycloalcanones ayant de 5 à 8 atomes de carbone dans le cycle (éventuellement substituées par des radicaux alkyles ayant de 1 à 4 atomes de carbone) ; les cycloalkylcétones ayant de 8 à 10 atomes de carbone ; les cétones aromatiques comme les diarylcétones, les arylalkylcétones, les aralkylarylcétones, les aralkylalkylcétones, ayant de 8 à 15 atomes de carbone.

Des exemples plus particuliers de cétones répondant aux critères énumérés ci-avant sont : l'acétone, la méthyléthylcétone, la méthyl n-propylcétone, la méthylisopropylcétone, la méthylisobutylcétone, la diéthylcétone, l'hexanone-2, l'hexanone-3, l'oxyde de mésityle, la cyclohexanone, les méthyl-2,3 ou 4 cyclohexanones, la benzophénone, l'acétophénone, la propiophénone la β-phénylpropiophénone, la phénylacétone.

L'acétone, la méthyléthylcétone, la cyclohexanone et l'acétophénone conviennent tout particulièrement bien comme solvants.

Le solvant que l'on choisit peut être constitué par une seule des cétones précitées ou par un mélange de plusieurs de ces composés.

L'oxydation du composé phénolique se déroule dans un milieu solvant à base de cétone qui renferme : d'une part des ions cuivreux ou cuivriques, et d'autre part des ions carboxylates ou phénates.

S'agissant des ions cuivreux ou cuivriques, on peut utiliser comme source d'ions divers sels de cuivre. Toutefois on fait appel de préférence aux halogénures de cuivre et en particulier aux chlorures cuivreux ou cuivriques. Le nitrate cuivrique convient également.

La quantité de catalyseur au cuivre, exprimée en nombre d'ions cuivre par mole de composé phénolique, peut varier dans de larges limites. En général, cette quantité représente de 0,01 à 5 ions cuivre par mole de phénol ; cependant, il n'est pas souhaitable d'un point de vue pratique d'avoir recours à des quantités de catalyseur introduisant plus de 1 ion cuivre par mole de phénol. On utilise de préférence de 0,02 à 1 ion cuivre par mole de phénol. Des quantités comprises entre 0,02 et 0,5 ion cuivre par mole de phénol conviennent tout particulièrement bien.

S'agissant de la source d'ions carboxylates, on fait appel en général à des carboxylates alcalins ou d'ammonium. Plus précisément, on fait appel aux sels des métaux alcalins ou d'ammonium dérivés : des acides mono ou polycarboxyliques aliphatiques, linéaires ou ramifiés, saturés ou éthyléniques, comportant de 2 à 10 atomes de carbone dans le cas des mono-acides et de 3 à 10 atomes de carbone dans le cas des poly-acides ; des acides cycloalcanecarboxyliques comportant de 5 à 12 atomes de carbone ; des acides aromatiques comportant de 7 à 12 atomes de carbone ; et des polymères d'acides éthyléniques. Outre les groupes hydroxycarbonyles, les acides précités peuvent comporter des atomes d'halogène ou des groupes fonctionnels tels que par exemple des groupes hydroxyles, étheroxydes.

A titre d'exemples spécifiques de sources d'ions carboxylates, on peut citer : les sels des métaux alcalins et d'ammonium dérivés des acides suivants : l'acide acétique, l'acide dichloroacétique, l'acide trichloroacétique, l'acide trifluoroacétique, l'acide propionique, l'acide lactique, l'acide citrique, l'acide butyrique, l'acide isobutyrique, l'acide pivalique, l'acide hexanoïque, l'acide décanoïque, l'acide stéarique, l'acide succinique, l'acide glutarique, l'acide adipique, l'acide acrylique, l'acide méthacrylique, l'acide cyclohexanecarboxylique, l'acide benzoïque, les acides nitrobenzoïques, les acides chlorobenzoïques, les polymères d'acides acrylique et méthacrylique.

Les sels de lithium, de sodium, de potassium et d'ammonium dérivés des acides acétique, dichloroacétique, trichloroacétique, trifluoroacétique, propionique, benzoïque et des polymères d'acides acrylique et méthacrylique conviennent particulièrement bien.

S'agissant de la source d'ions phénates, on fait appel en général à des phénates alcalins ou d'ammonium. La partie organique ou anionique de ces sels peut être issue avantageusement du composé phénolique soumis à l'oxydation mais elle peut provenir aussi de composés phénoliques autres que celui à oxyder. A noter dans ce cadre, qu'il peut être intéressant d'utiliser, pour la source d'ions phénates, des phénols qui présentent une constante d'ionisation pKa dans l'eau inférieure à celle du phénol à oxyder.

A titre d'exemple, lorsqu'on oxyde le phénol proprement dit, on peut citer comme sources d'ions phénates : les sels de lithium, de sodium, de potassium et d'ammonium dérivés : du phénol lui-même ; des ortho-, méta et paranitrophénols, des ortho-, méta et parachlorophénols, des dinitro-2,4 et -3,6 phénols ; du trichloro-2,4,6 phénol.

Les sels utilisés comme sources d'ions phénates peuvent être apportés à l'état préformé dans le

milieu d'oxydation ; mais ils peuvent aussi être préparés sans inconvénient « in situ », soit par action du métal alcalin ou de la base ammoniaque sur le phénol choisi pour engendrer les ions phénates, soit par action des hydrures de métaux alcalins sur ledit phénol.

La quantité de carboxylate ou de phénate alcalin et d'ammonium utilisée est choisie de manière qu'elle apporte dans le milieu d'oxydation de 0,05 à 5 ions carboxylates ou phénates par ion cuivre, et de préférence de 0,1 à 2 ions carboxylates ou phénates par ion cuivre.

La température à laquelle on conduit la réaction peut varier dans de larges limites. Des températures de 10 à 120 °C et de préférence de 30 à 80 °C conviennent bien.

L'oxydation est réalisée sous une pression partielle d'oxygène d'au moins 5 bars. Bien qu'il n'y ait pas de limite supérieure critique de la pression, en pratique, on ne recourt pas à des pressions partielles d'oxygène supérieures à 100 bars. L'oxydation est conduite de préférence sous une pression partielle d'oxygène comprise entre 5 et 50 bars.

Le gaz contenant de l'oxygène moléculaire peut être l'air ou de l'air appauvri ou enrichi en oxygène, ou des mélanges d'oxygène avec divers gaz inertes.

La concentration du composé phénolique à oxyder dans le solvant peut varier dans un large domaine de valeurs. Plus précisément, cette concentration, exprimée par le nombre de moles de composé phénolique par litre de solvant, peut être aussi basse que 0,1 mole/litre et on a constaté que des concentrations notablement plus élevées pouvant atteindre 5 moles/litre peuvent être utilisées sans inconvénient pour le taux de transformation du phénol à oxyder et le rendement en benzoquinone. En général, les concentrations du composé phénolique dans le solvant varient entre 0,5 et 4 moles/litre.

D'un point de vue pratique, la réaction est conduite dans un appareillage résistant à la pression, inerte vis-à-vis de la masse réactionnelle tel que les autoclaves en acier émaillé ou chemisé en tantale ou avec du téflon.

Les exemples suivants illustrent l'invention et montrent comment elle peut être mise en pratique.

### Exemple 1

Dans un autoclave en acier inoxydable de 140 ml dont les parois internes sont recouvertes de téflon, ledit autoclave étant agité par secousses, on charge : 0,075 mole de phénol ; 30 $cm^3$ de méthyléthylcétone ; 0,007 5 mole de chlorure cuivreux ; et 0,001 87 moles d'acétate de sodium. La concentration du phénol dans le solvant est de 2,5 moles/litre ; le nombre d'ions cuivre par mole de phénol est de 0,1 ; et le nombre d'ions acétate par ion cuivre est de 0,25. On ferme l'autoclave puis on le met en relation avec une source d'oxygène et on établit une pression de 21 bars d'oxygène. On met l'agitation en marche puis on porte la température à 60 °C. On maintient le contenu de l'autoclave sous agitation dans les conditions de pression et de température précitées pendant 4 heures. Après refroidissement, on dégaze la masse réactionnelle dans laquelle on dose par chromatographie en phase gazeuse le phénol non transformé et la para-benzoquinone.

Le taux de transformation du phénol (en abréviation : TT) s'élève dans ces conditions à 67,5 % et le rendement en benzoquinone par rapport au phénol transformé (en abréviation : RT) à 70 %.

### Exemples 2 à 8

On opère comme à l'exemple 1 en faisant varier les proportions de phénol, de chlorure cuivreux et d'acétate de sodium dans le milieu réactionnel. Les résultats sont rassemblés dans le tableau suivant :

| ex. | Phénol concentration dans solvant en moles/l | Chlorure cuivreux nombre d'ions cuivre/mole de phénol | Acétate de sodium nombre d'ions acétate/ion cuivre | TT % | RT % |
|---|---|---|---|---|---|
| 2 | 0,83 | 0,05 | 1 | 39 | 90 |
| 3 | 1,67 | 0,05 | 1 | 50 | 75 |
| 4 | 1,67 | 0,1 | 0,5 | 72 | 73 |
| 5 | 2,50 | 0,1 | 0,5 | 66 | 63,5 |
| 6 | 2,50 | 0,2 | 0,5 | 60,5 | 71 |
| 7 | 2,50 | 0,2 | 0,25 | 73 | 61 |
| 8 | 3,32 | 0,1 | 0,25 | 69,5 | 55 |

### Exemples 9 à 16

On opère comme à l'exemple 1 en faisant varier la nature de la source d'ions carboxylates, les autres conditions restant les mêmes, sauf indications contraires marquées entre parenthèses.

| ex. | Source d'ions carboxylates | TT % | RT % |
|---|---|---|---|
| 9 | $CH_3—CH_2—COONa$ | 63 | 71 |
| 10 | $CH_2Cl—COONa$ | 67 | 56 |
| 11 | $CCl_3$-COONa | 59,5 | 65 |
| 12 | $CF_3—COONa$ | 51 | 56,5 |
| 13 | $C_6H_5—COONa$ | 47 | 62,5 |
| 14 | $CH_3—COOLi$ | 67 | 70 |
| 15 | $CH_3—COOLi$ (0,4 ion acétate par ion cuivre) | 73 | 74 |
| 16 | $CH_3—COOK$ | 50 | 60 |

Exemple 17

On opère comme à l'exemple 1, mais en présence de chlorure cuivrique au lieu de chlorure cuivreux. Le taux de transformation du phénol (TT) s'élève à 58,5 % et le rendement en benzoquinone (RT) à 54,5 %.

Exemple 18

A titre de comparaison, on a répété l'exemple 1 mais en opérant en absence d'acétate de sodium. Le taux de transformation du phénol (TT) est de 10 % et le rendement en benzoquinone (RT) de 0 %.

Exemples 19 et 20

A titre de comparaison, on effectue l'oxydation du phénol en opérant dans le méthanol comme solvant en présence de chlorure cuivreux et de chlorure de lithium.

Selon l'exemple 19, on charge dans l'autoclave utilisé à l'exemple 1 : 0,025 mole de phénol ; 30 $cm^3$ de méthanol ; 0,001 25 mole de chlorure cuivreux ; et 0,002 5 mole de chlorure de lithium. La concentration du phénol dans le solvant est de 0,83 mole/litre ; le nombre d'ions cuivre par mole de phénol est de 0,05 ; et le nombre de moles de chlorure de lithium par ion cuivre est de 2. On opère comme indiqué à l'exemple 1 mais en travaillant sous une pression d'air de 100 bars.

Selon l'exemple 20, on charge dans l'autoclave utilisé à l'exemple 1 : 0,075 mole de phénol ; 30 $cm^3$ de méthanol ; 0,003 78 mole de chlorure cuivreux ; et 0,007 5 mole de chlorure de lithium. La concentration du phénol dans le solvant est augmentée par rapport à l'essai précédent jusqu'à 2,5 moles/litre ; le nombre d'ions cuivre par mole de phénol (0,05) et le nombre de moles de chlorure de lithium par ion cuivre (2) ne sont pas modifiés. On opère comme indiqué à l'exemple 1 en travaillant sous pression d'oxygène.

Les résultats ont été les suivants :

| | (TT) Taux de transformation du phénol | Rendement en benzoquinone (RT) |
|---|---|---|
| Exemple 19 | 67 % | 70 % |
| Exemple 20 | 89 % | 49 % |

Exemples 21 et 22

A titre de comparaison encore, on effectue l'oxydation du phénol en opérant dans l'acétonitrile comme solvant en présence de chlorure cuivreux uniquement.

Selon l'exemple 21, on charge dans l'autoclave utilisé à l'exemple 1 : 0,025 mole de phénol, 30 $cm^3$ d'acétonitrile et 0,002 5 mole de chlorure cuivreux. La concentration du phénol dans le solvant est de 0,83 mole/litre et le nombre d'ions cuivre par mole de phénol est de 0,1. On opère comme indiqué à l'exemple 1 mais en travaillant sous une pression d'air de 100 bars et à une température de 12 °C qui est celle donnant les meilleurs résultats au point de vue rendement en benzoquinone. La durée de réactior est de 4 heures.

Selon l'exemple 22, on charge dans l'autoclave utilisé à l'exemple 1 : 0,075 mole de phénol, 30 $cm^3$ d'acétonitrile et 0,007 5 mole de chlorure cuivreux. La concentration en phénol dans le solvant est augmentée par rapport à l'essai précédent jusqu'à 2,5 moles/litre, tandis que le nombre d'ions cuivre par mole de phénol (0,1) n'est pas modifié. On opère comme indiqué à l'exemple 1 en travaillant en relation avec une source d'oxygène à 21 bars, à 12 °C et pendant 4 heures.

Les résultats ont été les suivants :

| | Taux de transformation du phénol (TT) | Rendement en benzoquinone (RT) |
|---|---|---|
| Exemple 21 | 51 % | 73,5 % |
| Exemple 22 | 62 % | 48 % |

Exemples 23 à 27

On opère comme à l'exemple 1 en faisant varier la nature de la cétone utilisée comme solvant. Les autres conditions restent les mêmes, sauf indications contraires désignées entre parenthèses. Les résultats sont rassemblés dans le tableau suivant :

| ex. | Solvant | TT % | RT % |
|---|---|---|---|
| 23 | Acétone | 78 | 71,5 |
| 24 | Acétone (0,2 ion cuivre/mole de phénol) | 94 | 62 |
| 25 | Acétone (0,3 ion cuivre/mole de phénol) | 96,5 | 62 |
| 26 | Cyclohexanone (concentration du phénol : 0,83 mole/l ; 0,05 ion cuivre/mole de phénol ; et l ion acétate/ion cuivre) | 57,5 | 62,5 |
| 27 | Acétophénone | 53 | 57 |

Exemples 28 à 30

On opère comme à l'exemple 23 mais en présence d'acétate de lithium (avec 0,4 ion acétate par ion cuivre) à la place d'acétate de sodium et en utilisant diverses proportions d'ions cuivre par mole de phénol. Les résultats sont rassemblés dans le tableau suivant :

| ex. | Chlorure cuivreux ions cuivre/mole de phénol | Acétate de lithium ions acétate/ion cuivre | TT % | RT % |
|---|---|---|---|---|
| 28 | 0,1 | 0,4 | 81 | 74 |
| 29 | 0,15 | 0,4 | 95,5 | 76 |
| 30 | 0,2 | 0,4 | 100 | 70 |

Exemple 31

On opère comme à l'exemple 1 mais en faisant appel à l'orthocrésol à la place du phénol. La température d'oxydation est de 40 °C. Le taux de transformation de l'orthocrésol (TT) s'élève à 84 % et le rendement en méthyl-para-benzoquinone (RT) à 53,5 %.

Exemple 32

On opère comme à l'exemple 1 mais en remplaçant l'acétate de sodium par du phénate de sodium. Ledit phénate de sodium est préparé « in situ » par action du sodium métallique sur une partie du phénol à oxyder. Plus précisément, on charge dans l'autoclave : 0,075 mole de phénol, 30 $cm^3$ de méthyléthylcétone, et 0,001 87 mole de sodium métallique. On laisse alors réagir pendant 5 minutes, puis on ajoute 0,007 5 mole de chlorure cuivreux. On opère ensuite comme indiqué à l'exemple 1.

Le taux de transformation du phénol (TT) s'élève dans ces conditions à 52 % et le rendement en benzoquinone (RT) à 54,5 %.

Exemple 33

On a répété l'exemple 32 en opérant en présence de phénate de lithium au lieu de phénate de sodium. Pour ce faire, on charge à la place du sodium métallique, 0,002 25 mole d'hydrure de lithium.

Le taux de transformation du phénol (TT), s'élève à 65,5 % et le rendement en benzoquinone (RT) à 60 %.

Exemple 34

On a répété l'exemple 32 mais en opérant en présence de p-nitrophénate de lithium au lieu de phénate de sodium. Pour ce faire, on charge à côté du phénol et du solvant : 0,001 9 mole de p-nitrophénol et 0,001 9 mole d'hydrure de lithium. On laisse le milieu réagir pendant 5 minutes puis on ajoute le chlorure cuivreux.

Le taux de transformation du phénol (TT) s'élève à 61,5 % et le rendement en benzoquinone (RT) à 53 %.

Exemple 35

On a répété l'exemple 32 mais en opérant en présence de trichloro-2,4,6 phénate de lithium au lieu de phénate de sodium. Pour ce faire, on charge à côté du phénol et du solvant : 0,001 9 mole de trichloro-2,4,6 phénol et 0,001 9 mole d'hydrure de lithium. On laisse le milieu réagir pendant 5 minutes puis on ajoute le chlorure cuivreux.

Le taux de transformation du phénol (TT) s'élève à 64,5 % et le rendement en benzoquinone (RT) à 53,5 %.

**Revendications**

1. Procédé de préparation de para-benzoquinones par oxydation du phénol ou des phénols mono ou polysubstitués en ortho et/ou méta par l'oxygène moléculaire ou un gaz qui en contient en opérant dans un solvant organique en présence d'ions cuivreux ou cuivriques, caractérisé en ce que l'on opère dans une cétone comme solvant en présence, en outre, d'ions carboxylates ou phénates.

2. Procédé selon la revendication 1, caractérisé en ce que les ions carboxylates ou phénates sont sous forme de carboxylates ou de phénates alcalins ou d'ammonium.

3. Procédé selon l'une quelconque des revendications 1 à 2, caractérisé en ce que les phénols substitués sont des phénols de formule générale :

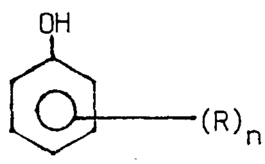

dans laquelle :

— R qui est situé en ortho ou méta par rapport au groupe hydroxyle représente : un radical alkyle comportant de 1 à 6 atomes de carbone (éventuellement substitué par un radical alkoxy comportant de 1 à 4 atomes de carbone) ; un radical aryle comportant de 6 à 15 atomes de carbone ; un radical arylalkyle comportant de 1 à 3 atomes de carbone dans le reste alkyle et 1 ou 2 noyaux benzéniques (condensés ou non) dans le reste aryle ;

— deux substituants R en position ortho et méta peuvent former ensemble un radical unique divalent alkylène ou alcénylène comportant de 5 à 7 atomes de carbone et le cas échéant 1 à 2 doubles liaisons éthyléniques (ledit radical étant éventuellement substitué par 1 à 3 radicaux alkyles ou alkoxy ayant de 1 à 4 atomes de carbone) ;

— n représente un nombre entier égal à 1, 2 ou 3.

4. Procédé selon l'une quelconque des revendications 1 à 3, caractérisé en ce que les cétones utilisables comme solvant sont : les cétones aliphatiques, saturées ou éthyléniques ayant de 3 à 10 atomes de carbone ; les cycloalcanones ayant de 5 à 8 atomes de carbone dans le cycle (éventuellement substituées par des radicaux alkyles ayant de 1 à 4 atomes de carbone) ; les cycloalkylcétones ayant de 8 à 10 atomes de carbone ; les cétones aromatiques comme les diarylcétones, les arylalkylcétones, les aralkylarylcétones, les aralkylalkylcétones, ayant de 8 à 15 atomes de carbone.

5. Procédé selon la revendication 4, caractérisé en ce que le solvant est : l'acétone, la méthyléthylcétone, la méthyl n-propylcétone, la méthylisopropylcétone, la méthylisobutylcétone, la diéthylcétone, l'hexanone-2 ; l'hexanone-3, l'oxyde de mésityle, la cyclohexanone, les méthyl-2, 3 ou 4 cyclohexanones, la benzophénone, l'acétophénone, la propiophénone, la β-phénylpropiophénone, la phénylacétone.

6. Procédé selon l'une quelconque des revendications 1 à 5, caractérisé en ce que les ions cuivre sont sous forme de chlorures cuivreux ou cuivriques.

7. Procédé selon l'une quelconque des revendications 1 à 6, caractérisé en ce que la quantité d'ions cuivre par mole de phénol est comprise entre 0,01 et 5.

8. Procédé selon l'une quelconque des revendications 1 à 7, caractérisé en ce que les ions carboxylates sont sous forme des sels de métaux alcalins et d'ammonium dérivés d'acides mono- ou polycarboxyliques aliphatiques, cycloalcanecarboxyliques ou aromatiques ou dérivés de polymères d'acides éthyléniques (lesdits acides pouvant éventuellement être substitués par des atomes d'halogène ou des groupes fonctionnels).

9. Procédé selon la revendication 8, caractérisé en ce que les ions carboxylates sont sous forme des sels de lithium, de sodium, de potassium, d'ammonium, des acides acétique, dichloroacétique, trichloroacétique, trifluoroacétique, propionique, benzoïque et des polymères d'acides acrylique et méthacrylique.

10. Procédé selon l'une quelconque des revendications 1 à 9, caractérisé en ce que les ions phénates sont sous forme des sels de métaux alcalins et d'ammonium dérivés du composé phénolique soumis à l'oxydation.

11. Procédé selon l'une quelconque des revendications 1 à 10, caractérisé en ce que la quantité d'ions carboxylates ou phénates par ion cuivre est comprise entre 0,05 et 5.

12. Procédé selon l'une quelconque des revendications 1 à 11, caractérisé en ce que la concentration du composé phénolique à oxyder dans le solvant est comprise entre 0,1 à 5 moles/litre.

13. Procédé selon l'une quelconque des revendications 1 à 12, caractérisé en ce que la température est comprise entre 10 et 120 °C et la pression partielle d'oxygène entre 5 et 100 bars.

**Claims**

1. Process for the preparation of para-benzoquinones by the oxidation of phenol, or of phenols which are monosubstituted or polysubstituted in the ortho- and/or metaposition, with molecular oxygen or a gas containing molecular oxygen, the reaction being carried out in an organic solvent, in the presence of cuprous or cupric ions, characterised in that the reaction is carried out in a ketone, as the solvent, and also in the presence of carboxylate or phenate ions.

2. Process according to Claim 1, characterised in that the carboxylate or phenate ions are in the form of alkali metal or ammonium carboxylates or phenates.

3. Process according to either one of Claims 1 and 2, characterised in that the substituted phenols are phenols of the general formula :

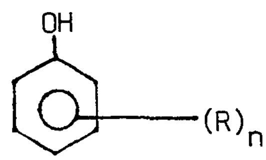

in which :

— R, which is located in the ortho- or meta-position relative to the hydroxyl group, represents : an alkyl radical which contains from 1 to 6 atoms (and is optionally substituted by an alkoxy radical containing from 1 to 4 carbon atoms), an aryl radical containing from 6 to 15 carbon atoms or an arylalkyl radical containing from 1 to 3 carbon atoms in the alkyl radical and 1 or 2 benzene nuclei (fused or non-fused) in the aryl radical, or

— two substituents R in the ortho- and meta-position can together form a single divalent alkylene or alkenylene radical containing from 5 to 7 carbon atoms and, if appropriate, 1 or 2 ethylenic double bonds (the said radical being optionally substituted by 1 to 3 alkyl or alkoxy radicals having from 1 to 4 carbon atoms), and

— n represents an integer equal to 1, 2 or 3.

4. Process according to any one of Claims 1 to 3, characterised in that the ketones which can be used as the solvent are : saturated or ethylenic aliphatic ketones having from 3 to 10 carbon atoms, cycloalkanones which have from 5 to 8 carbon atoms in the ring (and are optionally substituted by alkyl radicals having from 1 or 4 carbon atoms), cycloalkyl ketones having from 8 to 15 carbon atoms, and aromatic ketones having from 8 to 15 carbon atoms, such as diaryl ketones, aryl alkyl ketones, aralkyl aryl ketones and aralkyl alkyl ketones.

5. Process according to Claim 4, characterised in that the solvent is : acetone, methyl ethyl ketone, methyl n-propyl ketone, methyl isopropyl ketone, methyl isobutyl ketone, diethyl ketone, hexan-2-one, hexan-3-one, mesityl oxide, cyclohexanone, 2-, 3- or 4-methylcyclohexanone, benzophenone, acetophenone, propiophenone, β-phenylpropiophenone or phenylacetone.

6. Process according to any one of Claims 1 to 5, characterised in that the copper ions are in the form of cuprous or cupric chloride.

7. Process according to any one of Claims 1 to 6, characterised in that the amount of copper ions per mol of phenol is between 0.01 and 5.

8. Process according to any one of Claims 1 to 7, characterised in that the carboxylate ions are in the form of alkali metal and ammonium salts derived from aliphatic, cycloalkanecarboxylic or aromatic, monocarboxylic or polycarboxylic acids or derived from polymers of ethylenic acids (it being possible for the said acids to be optionally substituted by halogen atoms or functional groups).

9. Process according to Claim 8, characterised in that the carboxylate ions are in the form of the lithium, sodium, potassium and ammonium salts of acetic, dichloroacetic, trichloroacetic, trifluoroacetic, propionic and benzoic acids and of polymers of acrylic and methacrylic acids.

10. Process according to any one of Claims 1 to 9, characterised in that the phenate ions are in the form of the alkali metal and ammonium salts derived from the phenolic compound subjected to oxidation.

11. Process according to any one of Claims 1 to 10, characterised in that the amount of carboxylate or phenate ions per copper ion is between 0.05 and 5.

12. Process according to any one of Claims 1 to 11, characterised in that the concentration, in the

solvent, of the phenolic compound to be oxidised is between 0.1 and 5 mols/litre.

13. Process according to any one of Claims 1 to 12, characterised in that the temperature is between 10 and 120 °C and the oxygen partial pressure is between 5 and 100 bars.

**Ansprüche**

1. Verfahren zur Herstellung von para-Benzochinonen durch Oxydation von Phenol oder von in ortho und/oder meta-Stellung mono- oder polysubstituierten Phenolen durch molekularen Sauerstoff oder ein diesen enthaltendes Gas, wobei man in einem organischen Lösungsmittel in Gegenwart von Kupfer (I)- oder Kupfer (II)-ionen arbeitet, dadurch gekennzeichnet, daß man in einem Keton als Lösungsmittel in Gegenwart von Carboxylat- oder Phenolationen arbeitet.

2. Verfahren nach Anspruch 1, dadurch gekennzeichnet, daß die Carboxylat- oder Phenolationen in Form von Alkali- oder Ammoniumcarboxylaten oder -phenolaten vorliegen.

3. Verfahren nach einem der Ansprüche 1 bis 2, dadurch gekennzeichnet, daß die substituierten Phenole Phenole der allgemeinen Formel

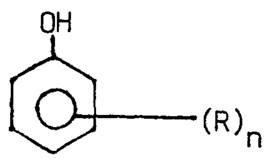

sind, worin

— R, das sich in ortho- oder meta-Stellung in bezug auf die Hydroxylgruppe befindet, bedeutet : einen Alkylrest mit 1 bis 6 Kohlenstoffatomen (gegebenenfalls substituiert durch einen Alkoxyrest mit 1 bis 4 Kohlenstoffatomen) ; einen Arylrest mit 6 bis 15 Kohlenstoffatomen ; einen Aralkylrest mit 1 bis 3 Kohlenstoffatomen im Alkylrest und 1 oder 2 (gegebenenfalls kondensierten) Benzolkernen im Arylrest ;

— zwei Substituenten R in ortho- und meta-Stellung zusammen einen zweiwertigen Alkylen- oder Alkenylenrest mit 5 bis 7 Kohlenstoffatomen und 1 bis 2 Äthylendoppelbindungen bilden können (der Rest kann gegebenenfalls durch 1 bis 3 Alkyl- oder Alkoxyreste mit 1 bis 4 Kohlenstoffatomen substituiert sein ;

— n eine ganze Zahl 1, 2 oder 3 bedeutet.

4. Verfahren nach einem der Ansprüche 1 bis 3, dadurch gekennzeichnet, daß die als Lösungsmittel einsetzbaren Ketone sind : gesättigte oder äthylenische aliphatische Ketone mit 3 bis 10 Kohlenstoffatomen ; Cycloalkanone mit 5 bis 8 Kohlenstoffatomen im Ring (gegebenenfalls substituiert durch Alkylreste mit 1 bis 4 Kohlenstoffatomen) ; Cycloalkylketone mit 8 bis 10 Kohlenstoffatomen ; aromatische Ketone, wie Diarylketone, Arylalkylketone, Aralkylarylketone, Aralkylalkylketone mit 8 bis 15 Kohlenstoffatomen.

5. Verfahren nach Anspruch 4, dadurch gekennzeichnet, daß das Lösungsmittel ist : Aceton, Methyläthylketon, Methyl-n-propylketon, Methylisopropylketon, Methylisobutylketon, Diäthylketon, Hexanon-(2), Hexanon-(3), Mesityloxid, Cyclohexanon, 2, 3 oder 4-Methylcyclohexanon, Benzophenon, Acetophenon, Propiophenon, β-Phenylpropiophenon, Phenylaceton.

6. Verfahren nach einem der Ansprüche 1 bis 5, dadurch gekennzeichnet, daß die Kupferionen in Form von Kupfer (I)- oder Kupfer (II)-chloriden vorliegen.

7. Verfahren nach einem der Ansprüche 1 bis 6, dadurch gekennzeichnet, daß die Menge an Kupferionen pro Mol Phenol zwischen 0,01 und 5 beträgt.

8. Verfahren nach einem der Ansprüche 1 bis 7, dadurch gekennzeichnet, daß die Carboxylationen in Form von Alkalimetall- und Ammoniumsalzen vorliegen, die von aliphatischen Mono- oder Polycarbonsäuren, Cycloalkancarbonsäuren oder aromatischen Säuren oder von polymeren Äthylensäuren (die Säuren können gegebenenfalls durch Halogenatome oder funktionelle Gruppen substituiert sein) abgeleitet sind.

9. Verfahren nach Anspruch 8, dadurch gekennzeichnet, daß die Carboxylationen in Form von Lithium-, Natrium-, Kalium-, Ammoniumsalzen von Essig-, Dichloressig-, Trichloressig-, Trifluoressig-, Propion-, Benzoesäure und polymeren Acryl- und Methacrylsäuren vorliegen.

10. Verfahren nach einem der Ansprüche 1 bis 9, dadurch gekennzeichnet, daß die Phenolationen in Form von der Oxydation unterworfenen Phenolverbindung abgeleiteten Alkalimetall- und Ammoniumsalzen vorliegen.

11. Verfahren nach einem der Ansprüche 1 bis 10, dadurch gekennzeichnet, daß die Menge von Carboxylat- oder Phenolationen pro Kupferion zwischen 0,05 bis 5 beträgt.

12. Verfahren nach einem der Ansprüche 1 bis 11, dadurch gekennzeichnet, daß die Konzentration der zu oxydierenden Phenolverbindung im Lösungsmittel zwischen 0,1 und 5 Mol/l beträgt.

13. Verfahren nach einem der Ansprüche 1 bis 12, dadurch gekennzeichnet, daß die Temperatur zwischen 10 und 120 °C und der Sauerstoffpartialdruck zwischen 5 und 100 bar beträgt.